# NOUVEAU FASCICULE DE BREVET EUROPEEN

(11) **EP 1 721 622 B2**
(45) Date de publication et mention de la décision concernant l'opposition: **28.11.2018**
(45) Mention de la délivrance du brevet: 29.10.2014
(21) Numéro de dépôt: 06007173.5
(22) Date de dépôt: 05.04.2006
(51) Int. Cl.: A61L 9/12, B60H 3/00, B60H 3/06

(54) **Dispositif de traitement de l'air**
Vorrichtung zur Luftbehandlung
Device for treating air

(30) Priorité: 22.04.2005 FR 0504070
(43) Date de publication de la demande: 15.11.2006
(73) Titulaire: VALEO SYSTEMES THERMIQUES, 78321 Le Mesnil St Denis Cedex (FR)
(72) Inventeur: Giraud, Frédéric, 78610 Le Perray en Yvelines (FR); Cornu, Laurence, 78730 Saint Arnoult en Yvelines (FR); Elliot, Gilles, 91080 Courcouronnes (FR)
(74) Mandataire: Metz, Gaëlle

(56) Documents cités:
- EP-A1- 1 082 969
- WO-A-03/077963
- WO-A-2005/014061
- WO-A1-03/077963
- WO-A1-2005/014061
- WO-A1-2005/087279
- US-A- 4 583 686
- US-A- 4 917 301
- US-A- 5 395 047
- US-A- 5 439 100
- US-A- 5 518 790
- US-A- 5 575 992
- US-A- 5 611 486
- US-A- 5 899 382
- US-A- 5 899 382
- US-A- 5 975 427
- US-A- 5 975 427
- Catalogue record -Air Wick Crystal Auto (Reckitt Benckiser), Mintel Catalogue record ID 340806
- Catalogue record -Air Wick Crstal Auto (Zoomed) Mintel Catalogue record ID 253864
- Catalogue record-Glade DeoSmile (S.C. Johnson), Mintel Catalogue Record ID 347713
- Catalogue record- Glade Clip-Ons (S.C. Johnson), Mintel Catalogue Record ID 259734

## Description

L'invention est du domaine de la désinfection, de la stérilisation et/ou de l'odorisation de l'air, notamment applicable aux installations de ventilation, de chauffage et/ou de climatisation, pour habitacle de véhicule automobile. Elle a pour objet une telle installation, comprenant un dispositif de traitement de l'air utilisant un agent traitant volatil logé. à l'intérieur d'un boîtier au moins partiellement perméable, pour notamment traiter l'air circulant au travers d'une telle installation.

On connaît des dispositifs pour traiter l'air utilisant un agent traitant volatil placé à l'intérieur d'un conteneur perméable, pour une diffusion .progressive de l'agent traitant à travers la paroi du conteneur sur une période donnée. Ce traitement est par exemple un traitement fongicide, anti-microbien ou odorant.

Selon une première solution connue, l'agent traitant est un liquide contenu à l'intérieur d'un sachet perméable au gaz et imperméable au liquide, lui-même emballé à l'intérieur d'un conteneur imperméable qui est retiré au moment de l'utilisation du dispositif. On pourra
par exemple se reporter au document US4961493 qui décrit un tel dispositif.

Inversement et selon une autre solution connue, l'agent traitant est un liquide contenu à l'intérieur d'un sachet imperméable, lui-même contenu dans un conteneur perméable. Lors de l'utilisation du dispositif, le sachet imperméable est rompu pour libérer l'agent traitant à l'intérieur du conteneur perméable. On pourra par exemple se reporter au document US5458244 qui décrit un tel dispositif. Il est également connu du document US5899382 un réservoir recouvert d'un film destiné à être retiré pour libérer un agent volatil.

Se pose le problème de l'accessibilité à ce type de conteneur dans un installation de ventilation, chauffage et/ou climatisation d'un véhicule neuf quand ce dernier est stocké sur parking pendant plusieurs mois avant d'être livré à son acquéreur. En effet, le conteneur est localisé à l'intérieur de l'installation, elle-même située sous la planche de bord et recouverte par des enjoliveurs plastiques. Si le conteneur est déclenché au moment de l'assemblage sur chaîne du véhicule, il aura alors une durée de vie réduite au moment de la livraison du véhicule à l'acquéreur final puisque les véhicules restent souvent parqués plusieurs mois avant la livraison, période pendant laquelle le conteneur se vide inutilement. Si l'on souhaite activer le conteneur au moment de la livraison, il faut alors démonter de nombreuses pièces telles que des éléments de la planche de bord pour atteindre l'endroit où est installé le conteneur. Ces opérations sont longues et risquées et ne sont donc pas compatibles avant une livraison du véhicule.

Ces contraintes se retrouvent quand le dispositif de traitement de l'air est combiné à un filtre pour le marché de la seconde monte automobile. On notera simplement à ce propos que le conteneur selon l'art antérieur n'est pas adapté pour être intégré à un filtre d'habitacle car son encombrement n'est pas étudié à cet effet et empiète grandement sur la surface filtrante du filtre.

Par ailleurs, on ne respecte pas les besoins des constructeurs automobiles en ce qui concerne la standardisation entre les produits de première monte et les produits de seconde monte de sorte à en faire baisser le coût de fabrication tout en respectant les caractéristiques techniques du véhicule tout au long de sa vie.

Enfin, le dispositif décrit dans l'art antérieur US5458244 n'est pas facile à mettre en oeuvre et ne permet pas d'être assuré quant à sa mise en fonctionnement.

Le but de la présente invention est donc de résoudre les inconvénients décrits ci-dessus principalement en proposant une installation de ventilation, chauffage et/ou climatisation d'un véhicule, comprenant un dispositif de traitement de l'air facile à fabriquer et qui peut être mis en fonctionnement aisément et ce à un instant déterminé, par exemple juste avant la livraison d'un véhicule neuf.

L'invention a donc pour objet une installation de ventilation, chauffage et/ou climatisation d'un véhicule, selon la revendication 1.

Selon une autre caractéristique de l'invention, la deuxième partie du film est repliée par-dessus la première partie avant déclenchement du moyen.

Selon encore une autre caractéristique de l'invention, le film est d'une épaisseur de l'ordre de 100 micromètres à 0,3 mm.

Selon encore une autre caractéristique de l'invention, la première partie est reliée au boîtier par collage, soudage ou thermoformage.

Selon encore une autre caractéristique de l'invention, la deuxième partie comprend une extrémité libre dépassant au-delà du dispositif.

L'installation selon l'invention comprend aussi un filtre d'habitacle pour véhicule automobile, comprenant un dispositif de traitement de l'air conforme à l'une quelconque des caractéristiques susmentionnées.

Selon l'invention, le filtre comprend un média filtrant plissé, le dispositif de traitement étant glissé dans l'un des plis du média filtrant.

Selon l'invention, le boîtier du dispositif est de forme en « V » et coopère parfaitement avec la forme plissée du média filtrant.

Selon l'invention, le dispositif de traitement de l'air est monté à l'intérieur de l'installation, dans une paroi à travers une ouverture.

Selon une autre caractéristique, la deuxième partie du dispositif de traitement est repliée par-dessus la première partie, longeant une des parois périphériques du boîtier, traversant l'ouverture et débouchant hors de l'installation.

Selon l'invention, l'installation comprenant un filtre d'habitacle conforme à l'une quelconque des caractéristiques susmentionnées, est caractérisée en ce qu'elle comprend une première paroi destinée à accueillir une extrémité du filtre d'habitacle et une seconde paroi présentant une ouverture au travers de laquelle est insérée le filtre.

Selon l'invention, la deuxième partie du moyen de déclencher l'émission de l'agent volatil passe au travers de l'ouverture et devient accessible via l'extrémité libre.

Un tout premier avantage de l'invention réside dans la possibilité de maintenir le dispositif dans une position inactive, c'est-à-dire de stockage, tout en ayant la possibilité d'activer l'émission de l'agent volatil à un moment déterminé et ce par un moyen aisé et facilement accessible, par l'utilisateur.

Un autre avantage réside dans la possibilité de standardiser le dispositif de traitement de l'air applicable à la première monte ou à la seconde des véhicules automobiles tout en ne limitant pas la surface filtrante du filtre d'habitacle.

D'autres caractéristiques, détails et avantages de l'invention ressortiront plus clairement à la lecture de la description donnée ci-après à titre indicatif en relation avec des dessins dans lesquels :
- les figures 1a à 1d sont des représentations du dispositif de traitement de l'air selon une première variante de l'invention,
- la figure 2 est une vue du dispositif de traitement avant mise en fonctionnement,
- la figure 3 est une vue similaire à la figure 2 dans laquelle le moyen de déclencher l'émission d'agent volatil est en cours de retrait,
- les figures 4a à 4d sont des représentations d'une variante du dispositif autre que l'invention,

Les dessins sont des représentations à caractère certain et pourront être utilisés pour définir l'invention le cas échéant.

Le but de la présente invention est de proposer un dispositif de traitement de l'air à partir d'un agent traitant volatil contenu à l'intérieur d'un boîtier perméable, qui offre une diffusion satisfaisante, stable et pérenne de l'agent volatil tout en évitant que cette diffusion se fasse pendant des périodes inappropriées.

Un tel dispositif est appliqué à une installation de ventilation, de chauffage et/ou de climatisation d'un véhicule, prenant en compte les contraintes d'initialisation et d'installation du dispositif à l'intérieur d'une telle installation.

Un tel dispositif est aussi destiné à être intégré à un filtre à air avant montage dans une installation de ventilation, chauffage et/ou climatisation. Ce filtre est du type à particules, à charbon actif ou une combinaison des deux. Dans le cas des filtres à air destinés à la seconde monte, on comprend que le dispositif de traitement de l'air doit être déclenché seulement lors du montage dans l'installation. Ceci évite que l'agent volatil migre prématurément vers le média filtrant et endommage ce dernier, notamment dans le cas d'un filtre à air utilisant du charbon actif.

L'agent traitant volatil peut être utilisé pour la désinfection, la stérilisation et/ou de l'odorisation de l'air qui circule à l'intérieur de l'installation de climatisation. Ce traitement est par exemple un traitement fongicide, anti-microbien qui utilise un agent traitant volatil du type allyl-isothiocyanate.

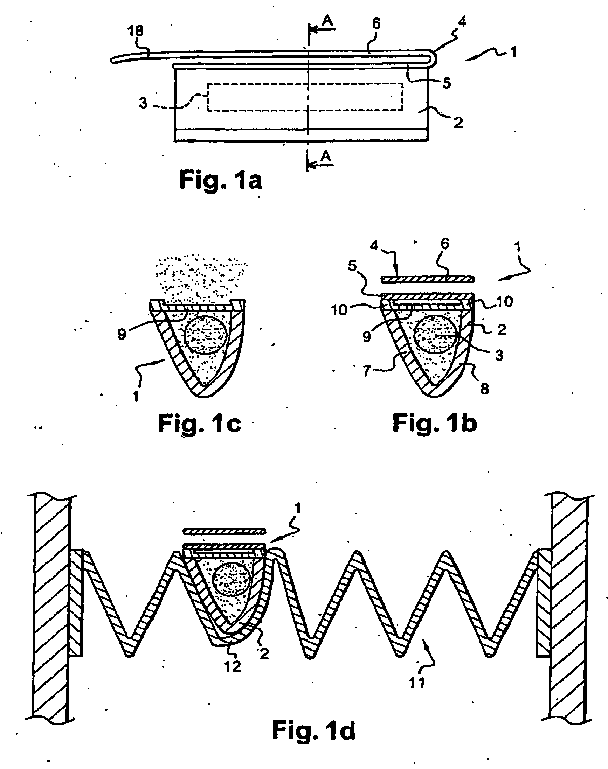

La figure 1 illustre le dispositif de traitement 1 de l'air dans une première variante de l'invention dans son état de stockage, c'est-à-dire avant déclenchement de l'émission d'agent volatil. Ce dispositif est constitué d'un boîtier 2 de 'forme allongée à l'intérieur duquel est glissé un élément 3 susceptible de contenir, maintenir ou stocker l'agent volatil. Cet élément peut être du type sac perméable contenant un liquide ou un gel actif ou du type corps cohésif spongieux absorbant l'agent volatil. La description qui suit sera faite en rapport avec un corps cohésif spongieux 3 mais il est bien entendu que toutes autres solutions aptes à contenir de manière perméable ou maintenir un agent volatil sont couvertes par l'objet de l'invention.

Ce boîtier au moins partiellement perméable est notamment formé d'une matière plastique, telle que par exemple du type polypropylène, éventuellement chargée de talc en proportion comprise entre 0% est 40%, et plus particulièrement de l'ordre de 20%, pour notamment une épaisseur de paroi comprise entre 0,5 mm et 2 mm.

Dans une forme avantageuse de l'invention, le diamètre interne du boîtier 2 est compris entre 9mm et 18mm mais plus particulièrement 11, 8mm, sa longueur est comprise entre 40mm et 200mm mais plus particulièrement 100mm et son épaisseur est comprise entre 0,85mm et 1,10mm mais plus particulièrement de l'ordre de 0,93mm ou 1,02mm.

Le boîtier 2 comprend sur sa partie supérieure un moyen 4 de déclencher l'émission de l'agent volatil vers l'extérieur du dispositif. On comprend que ce moyen de déclencher l'émission de l'agent volatil est une structure physique intégrée ou faisant partie, du dispositif de traitement de l'air.

Dans le cas de la première variante, ce moyen 4 est constitué d'un film ou d'une languette plastique aluminisée d'une épaisseur de l'ordre de 100 micromètres à 0, 3mm. Cette languette ou film est étanche et comprend une première partie 5 reliée au boîtier 2 par exemple par collage, soudage ou thermoformage et une deuxième partie 6 libre sensiblement repliée par-dessus la première partie et dont l'extrémité libre 18 dépasse au-delà du dispositif de traitement 1.

La figure 1b est une vue en coupe selon l'axe A-A représenté sur la figure 1a. On distingue la présence du corps cohésif spongieux 3, par exemple une mèche de forme tubulaire, insérée dans le volume intérieur du boîtier 2. Ce dernier prend une forme générale en « V » constituée par des parois imperméables 7 et 8. Cette forme en « V » est fermée par une paroi perméable d'épaisseur plus faible que les parois imperméables 7 et 8.

La paroi perméable est appelée surface active 9 car c'est la zone d'émission du dispositif de traitement 1 de l'air. La surface active 9 présente un bord périphérique 10 d'épaisseur plus importante que la surface active 9. Ce bord périphérique 10 est la zone de coopération, par exemple par collage, entre le moyen 4 de déclencher l'émission de l'agent volatil et le boîtier 2. La première partie 5 du film étant étanche et la fixation du film sur le bord périphérique 10 étant aussi étanche, on comprend que l'agent volatil ne peut pas sortir par la surface active 9. Le dispositif de traitement est donc représenté sur cette figure 1b en position de stockage, l'agent traitant volatil illustré par une multiplicité de points est contenu dans le volume intérieur du boîtier 2 délimité par les paroi imperméables 7 et 8 et par la première partie 5 du film.

Cela n'est pas le cas de l'illustration représentée à la figure 1c où on constate que le moyen 4 de déclencher l'émission d'agent volatil a été retiré. On permet ainsi à l'agent traitant de traverser la surface utile 9 pour se répandre à l'intérieur de l'installation de ventilation, chauffage et/ou climatisation comme ceci est illustré par le nuage de points représenté à l'extérieur ou autour du dispositif de traitement 1.

La surface active 9 est dimensionnée tant en ce qui concerne son épaisseur que sa largeur et sa longueur. Les critères qui déterminent ces dimensions sont imposés par la durée de vie souhaitée pour un tel dispositif de traitement ainsi que par la diffusion annuelle envisagée. A titre d'exemple, une diffusion annuelle de 45 mg/jour d'agent traitant, notamment de l'allyl-isothiocyanate, à une température constante de l'ordre de 40°C, peut être engendrée avec une épaisseur de la surface utile 9 de l'ordre de 1,0mm plus ou moins 20% pour une surface globale (longueur par largeur de la surface utile 9) de 3750mm2 plus ou moins 10%. On comprend donc qu'il s'agit d'une épaisseur maîtrisée ou prédéterminée.

La figure 1d est une représentation du dispositif de traitement 1 mis en situation sur un filtre d'habitacle 11 installé dans une installation de ventilation, chauffage et/ou climatisation illustrée ici partiellement. Le dispositif de traitement 1 est glissé dans un des plis du média filtrant 12 dans un état où le moyen 4 de déclencher l'émission d'agent volatil n'est pas mis en oeuvre. La forme en « V » du boîtier 2 coopère parfaitement avec la forme plissée du média filtrant 12 si bien que le dispositif 1 s'intègre complètement au filtre habitacle. Le dispositif de traitement 1 est bien entendu relié fixement au média 12 par des moyens appropriés.

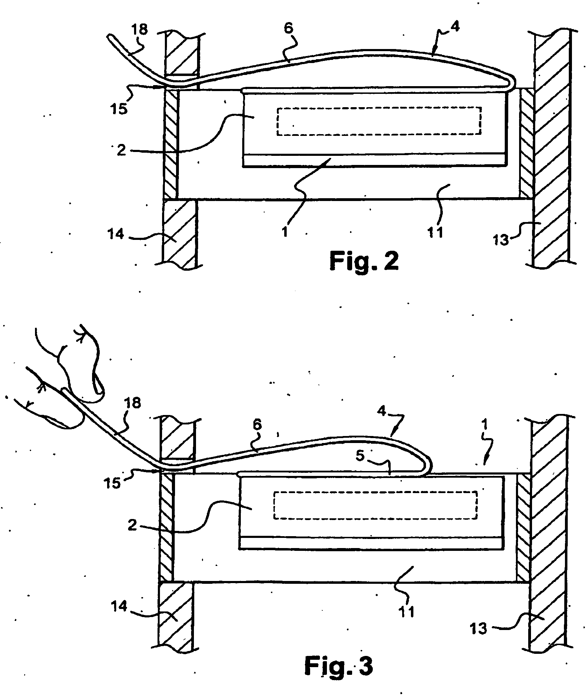

La figure 2 est une vue de gauche de la figure 1d. Le filtre habitacle 11 est monté transversalement dans l'installation de ventilation. Cette dernière présente d'une part une première paroi 13 qui sert de fond et qui accueille une extrémité du filtre d'habitacle 11 et d'autre part une seconde paroi 14 qui présente une ouverture 15 au travers de laquelle est inséré le filtre. L'étanchéité à l'air peut être pratiquée directement entre la seconde paroi 14 de l'installation et le filtre habitacle 11 mais celle-ci peut aussi être réalisée en recouvrant l'ouverture 15 par une trappe (non représentée) fixée sur la seconde paroi 14.

Le dispositif de traitement 1 est en état de stockage puisque le moyen 4 de déclenchement de l'émission de l'agent volatile est fixé de manière étanche sur le boîtier 2. La deuxième partie 6 du moyen 4 passe au travers de l'ouverture 15 et devient donc accessible via l'extrémité libre 18 pour un utilisateur ou pour un garagiste qui s'apprête à livrer le véhicule équipé de ce dispositif de traitement de l'air. L'accès à l'extrémité libre 18 de la deuxième partie 6 dépend de la localisation du dispositif de traitement 1 dans l'installation de climatisation. Quand celui-ci est distinct du filtre, l'extrémité de la deuxième partie 6 peut être rendue accessible en dépassant de l'installation de climatisation par l'ouverture nécessaire à l'insertion du dispositif de traitement. L'accès peut être opéré par la boîte à gants, par la zone pied avant du véhicule ou par le séparateur d'eau. Quand le dispositif de traitement de l'air est intégré au filtre d'habitacle, l'accès à l'extrémité libre 18 de la languette ou moyen 4 de déclencher l'émission d'agent volatil est conditionné par le positionnement du filtre. Celui-ci étant une pièce vouée à être remplacée relativement souvent, son accès est toujours étudié de manière aisée. En conséquence, il en sera de même pour l'accès à l'extrémité libre 18 de la deuxième partie 6 du film ou languette.

La figure 3 montre le dispositif de traitement 1 de l'air tel que décrit à la figure 2. Le moyen 4 de déclencher l'émission de l'agent volatil est illustré en cours de retrait. L'utilisateur tire sur l'extrémité libre 18 de la deuxième partie 6, le film est guidé pendant son retrait par le bord périphérique de l'ouverture 15 de sorte à exercer un effort d'arrachement sur la première partie 5 du film. Ce dernier roule donc sur lui-même jusqu'à son arrachement complet. A ce stade, le dispositif de traitement de l'air est mis en fonctionnement par enclenchement du moyen 4 d'émission de l'agent volatil qui libère la surface utile 9.

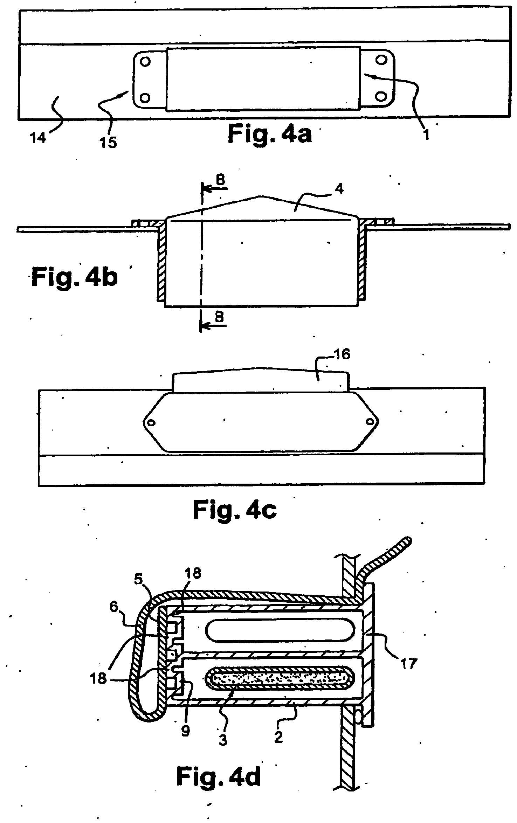

Les figures 4a à 4d illustrent une variante autre que l'invention qui répond à une contrainte de montage dans une installation exiguë Le dispositif de traitement 1 de l'air est donc plus large que long ce qui impose d'adapter le moyen 4 de déclencher l'émission d'agent volatil.

Le dispositif de traitement 1 de l'air est donc monté dans une paroi 14 à travers une ouverture 15. La figure 4a montre le dispositif de traitement 1 vu de l'intérieur de l'installation alors que le moyen 4 de déclencher l'émission d'agent volatil bloque la surface active. La figure 4b est une vue de dessus de la figure 4a alors que la figure 4c est une illustration du même dispositif vu de l'extérieur de l'installation. L'extrémité libre 18 de la deuxième partie 6 du film dépasse à l'extérieur et peut donc être attrapée par un utilisateur.

La figure 4d est une coupe selon l'axe B-B représenté sur la figure 4b. Le dispositif de traitement 1 de l'air comprend un élément de diffusion 3 logé dans un boîtier 2 de forme rectangulaire. La surface utile 9 est localisée à l'extrémité du boîtier 2 opposée à une planque de fermeture 17 qui obstrue l'ouverture 15 pratiquée dans l'installation.

Avantageusement, la surface active 9 présente une forme crénelée de sorte à augmenter la surface d'émission de l'agent volatil tout en conservant un volume acceptable.

Les extrémités 18 de la surface active crénelée coopèrent avec la première partie 5 du moyen de déclencher l'émission d'agent volatil. La deuxième partie 6 est quant à elle repliée par-dessus la première partie 5 puis longe une des parois périphériques du boîtier 2 pour finalement traverser l'ouverture 15 et déboucher hors de l'installation. La longueur nécessaire au retrait du moyen 4 de déclencher l'émission d'agent volatil est plus courte que pour la première variante de l'invention tandis que l'effort nécessaire à ce retrait sera plus important.

## Revendications

1. Installation de ventilation, chauffage et/ou climatisation d'un véhicule, **caractérisée en ce qu'**elle comprend, un dispositif de traitement (1) de l'air utilisant un agent volatil contenu à l'intérieur d'un boîtier (2) au moins partiellement perméable, comprenant un moyen (4) de déclencher l'émission de l'agent volatil, la perméabilité du boîtier (2) étant assurée par une surface active (9) dudit boîtier traversé par l'agent traitant volatil, la surface active (9) étant matérialisée par une paroi perméable d'épaisseur plus faible que les autres parois du boîtier (2), le moyen (4) de déclencher l'émission de l'agent volatil coopérant avec ladite surface active (9) pour maintenir étanche cette dernière avant le déclenchement du dispositif, **caractérisé en ce que** le moyen (4) de déclencher l'émission de l'agent volatil est un film imperméable constitué d'une première partie (5) recouvrant ladite surface active (9) et fixé de manière étanche à la périphérie de la zone active et d'une deuxième partie (6) libre accessible à l'utilisateur pour déclencher l'émission de l'agent volatil, ladite installation comprenant un filtre d'habitacle (11) à air de type à particules, à charbon actif ou une combinaison des deux pour véhicule automobile, monté transversalement dans ladite installation, ladite installation comprenant une première paroi (13) destinée à accueillir une extrémité du filtre d'habitacle (11), le filtre d'habitacle (11) étant inséré au travers d'une ouverture (15) d'une seconde paroi (14) de l'installation, la deuxième partie (6) du moyen (4) de déclencher l'émission de l'agent volatil passant au travers de l'ouverture (15) et devenant accessible via l'extrémité libre (18), le filtre d'habitacle (11) comprenant un média filtrant (12) plissé, le dispositif de traitement (1) étant glissé dans l'un des plis du média filtrant (12), le boîtier (2) du dispositif de traitement (1) étant de forme en « V » et coopérant parfaitement avec la forme plissée du média filtrant (12), le dispositif de traitement (1) étant relié fixement au média (12) par des moyens appropriés.

2. Installation selon la revendication 1, dans laquelle la deuxième partie (6) du film est repliée par-dessus la première partie (5) avant déclenchement du moyen (4).

3. Installation selon la revendication 2, **caractérisée en ce que** le film est d'une épaisseur de l'ordre de 100 micromètres à 0,3 mm, la première partie (5) est reliée au boîtier (2) par collage, soudage ou thermoformage, et **en ce que** la deuxième partie (6) comprend une extrémité libre (18) dépassant au-delà du dispositif (1).

## Patentansprüche

1. Belüftungs-, Heiz- und/oder Klimaanlage eines Fahrzeugs, **dadurch gekennzeichnet, dass** sie eine Luftbehandlungsvorrichtung (1) aufweist, die ein flüchtiges Mittel verwendet, das im Inneren eines Gehäuses (2), das mindestens teilweise durchlässig ist, enthalten ist, die ein Mittel (4) zum Auslösen des Abgebens des flüchtigen Mittels aufweist, wobei die Durchlässigkeit des Gehäuses (2) durch eine aktive Oberfläche (9) des Gehäuses, die von dem flüchtigen Behandlungsmittel durchquert wird, sichergestellt ist, wobei die aktive Oberfläche (9) von einer durchlässigen Wand mit geringerer Stärke als die anderen Wände des Gehäuses (2) verkörpert wird, wobei das Auslösemittel (4) des Abgebens des flüchtigen Mittels mit der aktiven Oberfläche (9) zusammenwirkt, um diese Letztere vor dem Auslösen der Vorrichtung dicht zu erhalten, **dadurch gekennzeichnet, dass** das Auslösemittel (4) des Abgebens des flüchtigen Mittels eine undurchlässige Folie ist, die aus einem ersten Teil (5) besteht, der die aktive Oberfläche (9) abdeckt und dicht an dem Umfang der aktiven Zone befestigt ist, und aus einem freien zweiten Teil (6), der für den Benutzer zum Auslösen des Abgebens des flüchtigen Mittels zugänglich ist, Anlage wobei die ein Insassenraum-Luftfilter (11) mit Partikeln, Aktivkohle oder einer Kombination beider für Kraftfahrzeuge aufweist, das in der Anlage quer installiert ist, wobei die Anlage eine erste Wand (13) aufweist, die dazu bestimmt ist, ein Ende des Insassenraum-Luftfilters (11) aufzunehmen, wobei das Insassenraum-Luftfilter (11) durch eine Öffnung (15) einer zweiten Wand (14) der Anlage eingefügt wird, wobei der zweite Teil (6) des Auslösemittels (4) des Abgebens des flüchtigen Mittels durch die Öffnung (15) durchgeht und über das freie Ende (18) zugänglich wird, wobei das Insassenraum-Luftfilter (11) einen gefältelten Filterträger (12) aufweist, wobei die Behandlungsvorrichtung (1) in eine der Falten des Filterträgers (12) geschoben wird, wobei das Gehäuse (2) der Behandlungsvorrichtung (1) "V"-Form hat und perfekt mit der gefältelten Form des Filterträgers (12) zusammenwirkt, wobei die Behandlungsvorrichtung (1) fest mit dem Träger (12) durch geeignete Mittel verbunden ist.

2. Anlage nach Anspruch 1, bei der der zweite Teil (6) der Folie über den ersten Teil (5) vor dem Auslösen des Mittels (4) zurückgefaltet wird.

3. Anlage nach Anspruch 2, **dadurch gekennzeichnet, dass** die Folie eine Stärke in der Größenordnung von 100 Mikrometer bis 0,3 mm aufweist, der erste Teil (5) mit dem Gehäuse (2) durch Kleben, Schweißen oder Wärmeformen verbunden ist, und dass der zweite Teil (6) ein freies Ende (18) aufweist, das über die Vorrichtung (1) hinausreicht.

## Claims

1. Vehicle heating, ventilation and/or air-conditioning installation, **characterized in that** it comprises an air-treatment device (1) using a volatile agent contained inside an at least partially permeable casing (2), comprising a means (4) of triggering the emission of the volatile agent, the permeability of the casing (2) being afforded by an active surface (9) of the said casing through which the volatile treatment agent passes, the active surface (9) being embodied by a permeable wall of lesser thickness than the other walls of the casing (2), the means (4) of triggering the emission of volatile agent collaborating with the said active surface (9) to keep the latter impervious until the device is triggered, **characterized in that** the means (4) of triggering the emission of the volatile agent is an impermeable film consisting of a first part (5) covering the said active surface (9) and fixed in a fluidtight manner to the periphery of the active zone, and of a free second part (6) accessible to the user to trigger the emission of the volatile agent, the said installation comprising a cabin air filter (11) of the particle filter type, active charcoal type or a combination of both, for a motor vehicle, mounted transversely in the said installation, the said installation comprising a first wall (13) intended to accommodate one end of the cabin filter (11), the cabin filter (11) being inserted through an opening (15) in a second wall (14) of the installation, the second part (6) of the means (4) of triggering the emission of volatile agent passing through the opening (15) and becoming accessible via the free end (18), the cabin filter (11) comprising a pleated filter medium (12), the treatment device (1) being slipped into one of the folds of the filter medium (12), the casing (2) of the treatment device (1) being in the shape of a V and collaborating perfectly with the pleated shape of the filter medium (12), the treatment device (1) being fixedly connected to the medium (12) by appropriate means.

2. Installation according to Claim 1, in which the second part (6) of the film is folded on top of the first part (5) before the means (4) is triggered.

3. Installation according to Claim 2, **characterized in that** the film is of a thickness of the order of 100 micrometres to 0.3 mm, the first part (5) is attached to the casing (2) by bonding, welding or thermoforming, and **in that** the second part (6) comprises a free end (18) extending beyond the device (1).
